(19) Europäisches Patentamt
European Patent Office
Office européen des brevets

(11) EP 1 510 817 A1

(12) EUROPEAN PATENT APPLICATION

(43) Date of publication:
02.03.2005 Bulletin 2005/09

(51) Int Cl.7: G01N 31/22, G01N 21/64

(21) Application number: 04018192.7

(22) Date of filing: 31.07.2004

(84) Designated Contracting States:
AT BE BG CH CY CZ DE DK EE ES FI FR GB GR HU IE IT LI LU MC NL PL PT RO SE SI SK TR
Designated Extension States:
AL HR LT LV MK

(30) Priority: 12.08.2003 US 638393

(71) Applicant: Becton Dickinson and Company
Franklin Lakes, NJ 07414-1880 (US)

(72) Inventors:
• Yeh, Ming-Hsiung
  New Freedom Pennsylvania 17349 (US)

• Keith, Steven
  Chapel Hill North Carolina 27514 (US)
• Rowley, Jon
  Chapel Hill North Carolina 27517 (US)
• Heidaran, Mohammad
  North Carolina 27513 (US)
• Hemperly, John
  Apex North Carolina 27502 (US)

(74) Representative:
von Kreisler, Alek, Dipl.-Chem. et al
Patentanwälte,
von Kreisler-Selting-Werner,
Bahnhofsvorplatz 1 (Deichmannhaus)
50667 Köln (DE)

(54) Microencapsulation of oxygen-sensing particles

(57) The present invention relates to compositions comprising a core and a hydrophobic coating material surrounding the core. The core comprises at least one oxygen-sensing particle. The present invention also relates to methods of detecting and monitoring oxygen in a sample using the microencapsulated oxygen-sensing particles.

Figure 1a

Oxygen sensitivity

- oil, no sulfite
- oil, plus sulfite
- no oil, no sulfite
- no oil, plus sulfite

Figure 1b

## Description

### Background of the Invention

*Field of the Invention*

The present invention relates to compositions comprising a core and a hydrophobic coating material surrounding the core. The core comprises at least one oxygen-sensing particle. The present invention also relates to methods of detecting and monitoring oxygen in a sample using the microencapsulated oxygen-sensing particles.

*Background of the Invention*

Conventional methods for monitoring cell growth, such as measuring cellular DNA with fluorescent dye, measuring cell metabolism or directly counting cells, is invasive, disruptive and may result in non-reproducible values. These end point assays are labor-intensive, and the sample requirements are expensive because different samples are needed at each time point. Thus, end point assays are not useful for monitoring cell growth over time in a high throughput manner.

Another approach to cell culture progress involves the use of oxygen sensors. These devices provide an effective way of monitoring cell growth in cell culture. The oxygen sensor directly measures cell metabolism, which gives an indirect measure of cell growth. Solid-state, fluorescence-based oxygen sensors are highly sensitive, selective and affordable. Oxygen sensors can provide a non-invasive real time measurement of cell growth in cell culture.

Typically, oxygen sensors are based on fluorescent dye crystals that exhibit strong luminescence upon irradiation. The luminescent properties of the fluorescent dye crystals may be efficiently quenched by oxygen, which results in a change in the luminescence signal directly related to the oxygen partial pressure in the environment. Organic ruthenium (II) complexes are popular oxygen sensor dyes owing to their high-quantum yield luminescence, high selectivity, good photostability, and relatively long lifetime. However, in a hydrophilic environment, the response of these oxygen-sensing particles is greatly diminished, if not completely ablated.

To overcome this problem of diminishing sensitivity in hydrophilic environments, the oxygen-sensing particles are dispersed in a hydrophobic polymeric fluid and then cured onto a flat surface ("slab") such as the bottom of a microtiter well. However, this type of slab configuration is not very responsive to changes in oxygen. Furthermore, this type of slab configuration is not amenable for use in three-dimensional cell culture settings such as hydrogel.

Thus, there is a need for a more versatile, responsive oxygen-sensing particle whose signal will not be diminished in a hydrophilic environment.

### Summary of the Invention

The present invention relates to compositions comprising a core and a hydrophobic coating material surrounding the core. The core comprises at least one oxygen-sensing particle. The present invention also relates to methods of detecting and monitoring oxygen in a sample using the microencapsulated oxygen-sensing particles.

### Brief Description of the Drawings

Figure 1 is a diagram showing the oxygen responses of the ruthenium dye-adsorbed silica gel particles to oxygen concentration change.

Figure 2 is a micrograph of silicone rubber-encapsulated oxygen-sensing particles.

Figure 3 shows the responsiveness to changes in oxygen of the MOSPs of the current invention embedded in hydrogel.

Figure 4 shows microencapsulated oxygen sensing particles embedded within a 3D matrix modified with fibronectin to support cell growth.

Figure 5 shows an example of a possible array comprising the compositions of the current invention.

### Detailed Description of the Invention

The present invention relates to compositions for detecting and monitoring oxygen in a sample comprising a core and a hydrophobic coating material surrounding the core. The core comprises at least one oxygen-sensing particle. The core comprises at least one oxygen-sensing particle but the core may also contain more than one oxygen sensing particle. These oxygen-sensing particles are microencapsulated by the hydrophobic coating material, thereby forming microencapsulated oxygen-sensing particles (MOSPs).

As used herein, "oxygen-sensing particles" are chemical entities that emit or generate a detectable signal in the presence or absence of oxygen. As used herein, the terms "particle", "granule" and "crystal" are used interchangeably. In one embodiment of the present invention, the at least one oxygen-sensing particle that comprises the core is luminescent. Typically, the generated or emitted signal from the luminescent oxygen-sensing particles may be detected with or without the aid of equipment such as, but not limited to, a spectrophotometer.

The presence of oxygen changes the rate at which the electrons return to the ground state. Consequently, it is possible to determine the oxygen concentration by observing the temporal activity of the luminophore. The relationship between the average decay time (t) and the oxygen concentration $[O_2]$ is also described (in the ideal case) by the Stem-Volmer equation:

$$[O_2] = (t_0/t-1) \cdot 1/K_{sv}$$

where $K_{sv}$ is the Stern-Volmer constant, and $t_0$ is the luminescence decay time in the absence of oxygen.

In another embodiment of the present invention, the emitted light signal from the luminescent oxygen-sensing particle is diminished in the presence of oxygen. Thus, in one specific embodiment of the present invention, the light signal from the luminescent oxygen-sensing compound can be quenched upon exposure to oxygen at a concentration that is ordinarily found in, for example, cell cultures (generally 0.4%). As used herein, an "inhibitory amount of oxygen" is a level of oxygen that diminishes the detectable signal as compared to the signal generated when no detectable oxygen is present. The diminishment of the detectable signal may be partial or complete.

As understood in the art, a "luminescent particle" is a particle capable of emitting light energy upon the application of energy to the particle. As used herein, "luminescence" includes, but is not limited to, fluorescence, time-resolved fluorescence, fluorescence lifetime, photoluminescence, phosphorescence, chemiluminescence, bioluminescence, electroluminescence, radioluminescence, triboluminescence, thermoluminescence and optically stimulated luminescence.

Examples of luminescent oxygen-sensing particles include but are not limited to any salt of tris-4,7-diphenyl-1,10-phenanthroline ruthenium (II), any salt of tris-2,2'-bipyridyl-ruthenium (II), any salt of tris-1,7-diphenyl-1,10 phenanthroline ruthenium (II), and 9,10-diphenyl anthracene. Luminescent particles can also include platinum (II) octaethyl porphyrin complexes, palladium (II) octaethyl porphyrin complexes, palladium-meso-tetra(4-carboxyphenyl) porphine, palladium-meso-tetra(4-carboxyphenyl) porphyrin dendrimer and palladium-meso-tetra(4-carboxyphenyl) tetrabenzoporphyrin dendrimer.

Examples of salts of tris-4,7-diphenyl-1,10-phenanthroline ruthenium (II) include, but are not limited to, tris-4,7-diphenyl-1,10-phenanthroline ruthenium (II) dichloride pentahydrate, tris-4,7-diphenyl-1,10-phenanthroline ruthenium (III) trichloride, tris-4,7-diphenyl-1,10-phenanthroline ruthenium (II) diperchlorate and tris-4,7-diphenyl-1,10-phenanthroline ruthenium hexafluorophosphate.

An example of a salt of tris-2,2'-bipyridyl-ruthenium (II) includes, but is not limited to, tris-2,2'-bipyridyl-ruthenium (II) chloride hexahydrate.

An example of a salt of tris-1,7-diphenyl-1,10 phenanthroline ruthenium (II) includes, but is not limited to, tris-1,7-diphenyl-1,10 phenanthroline ruthenium (II) dichloride.

In the compositions of the present invention, the oxygen-sensing particle core is coated with a hydrophobic coating material that surrounds the core. The hydrophobic coating material creates a microencapsulated oxygen-sensing particle (MOSP). The hydrophobic coating material is designed to protect the oxygen-sensing particle from hydrophilic environments, which may interfere with the signal generated or emitted by the oxygen-sensing particle. The hydrophobic coating material should also be permeable or semi-permeable to oxygen, such that the oxygen-sensing particles in the cores of the compositions of the present invention may still detect oxygen. Thus, any hydrophobic coating material that is permeable or semi-permeable to oxygen is well within the contemplated scope of the invention described herein.

As used herein, a "hydrophobic coating material" is any kind of entity that will not readily dissolve in a hydrophilic environment. In the context of the present invention, the coating should surround and encapsulate the oxygen-sensing granules or particles such that a hydrophilic substance cannot penetrate the coating material and reach the oxygen-sensing particle.

In one embodiment of the present invention, the hydrophobic coating material is a polymer or dendrimer. The terms "polymer" and "dendrimer" are used as one of ordinary skill in the art would recognize these terms. Examples of hydrophobic coating polymers include, but are not limited to, functional polydimethylsiloxane, silicone rubber, polytetrafluoroethylene (PTFE), polysterene, and mineral oil. Examples of specific coating material include but are not limited to, platinum curable two-part vinyl functionalized polydimethysiloxanes, hydrido functionalized polydimethylsiloxanes, alkoxyl functionalized polydimethylsiloxanes and acetoxyl functionalized polydimethylsiloxanes.

Specifically, functional polydimethylsiloxanes include, but are not limited to, vinyl functionalized polydimethylsiloxanes, hydrido functionalized polydimethylsiloxanes, alkoxyl functionalized polydimethylsiloxanes and acetoxyl functionalized polydimethylsiloxanes.

In another embodiment, the core of the MOSPs further comprises a carrier molecule. In one embodiment, the carrier molecule is a molecule with which the oxygen-sensing particles are admixed. In this embodiment, the carrier and the oxygen-sensing particle can simply be admixed and the hydrophobic coating material can be added to the granulate mixture. In another embodiment, the carrier and the oxygen-sensing particles will attach to each other. In this embodiment, the attachment of the oxygen-sensing particle to the carrier can be by any means, including but not limited to, adsorption, covalent binding, non-covalent binding, ionic bonding, hydrogen bonding, polar forces, and metallic bonding. Examples of carrier molecules include, but are not limited to, silica and polystyrene.

Furthermore, the carrier molecules used in the compositions of the present invention can themselves be modified. Modifications include, but are not limited to, bonding or attaching a long hydrocarbon chain covalently attached to the surface of, for example, silica particle carriers. The long hydrocarbon chain may contain a sufficient number of carbons to render the carrier more hydrophobic. For example, $C_{18}$ works well with silica particles. The silica particles having $C_{18}$ covalently attached thereon may further be coated with mineral oil, which has shown fast response and high sensitivity to oxygen concentration. Silica particles covalently attached to the long hydrophobic hydrocarbon chain may be commercially available.

In yet another embodiment of the present invention, one or more additional coatings can be applied to encapsulate the MOSPs (encapsulated MOSPs). As used herein, "MOSP" is used to mean an encapsulated MOSP in addition to a MOSP. The additional layers of coating will form concentric coatings around the core oxygen-sensing particle. The coating material for the additional layers can be hydrophobic or hydrophilic in nature, provided they are permeable to oxygen. Furthermore, these additional layers should encapsulate the first hydrophobic coating material, which is, in turn, encapsulating the oxygen-sensing core particles.

In still another embodiment, any of the compositions of the current invention may further comprise a matrix. As used herein, a "matrix" is a solid or semi-solid structure wherein the MOSPs can be incorporated onto or into the matrix. Examples of solid matrices include, but are not limited to, glass, nylon, plastic, polystyrene, polypropylene, polycarbonate, polymethacrylate, polyvinylchloride and latex.

Examples of a semi-solid hydrogel matrices include, but are not limited, to agarose, ionically crosslinked alginate, modified alginate (as described in Lee, K.Y. *et al.,* Macromolecules, 33:4219-4294 (2000), which is hereby incorporated by reference), agarose, cellulose, dextran, carboxymethyl cellulose, carboxymethyl dextran, collagens, matrigel, hyaluronic acid, modified hyaluronic acids, polyacrylimide, polyethylene glycol (PEG), polyvinylalcohol (PVA) and poly methylmethacrylate (PMMA), and combinations thereof. As used herein, "hydrogel" refers to a semisolid composition constituting water and dissolved, dispersed, and/or crosslinked polymers.

Additionally, the hydrogel matrix may or may not have been further processed. Examples of further processing of the hydrogel matrix include, but are not limited to, lyophilization, drying, leaching, centrifugation or spinning. Other methods of making and modifying hydrogel matrices are disclosed in United States Serial No. 10/259,817, filed on September 30, 2002, which is hereby incorporated by reference.

In another embodiment of the present invention, the MOSPs may be placed in or on a so-called three-dimensional matrix (3D matrix) for cell culture and tissue engineering. For example, the MOSPs may be placed in the matrix at the synthesis stage of the matrix. Additionally, the MOSPs may be added to or dispersed into a hydrogel solution, which is then crosslinked and lyophilized to make a 3D matrix comprising MOSPs for cell culture. Alternatively, the MOSPs may be seeded in or on the matrix, either alone or with cells to be cultured in the matrix. The cell types that can be cultured on the matrix include, but are not limited to animal, plant, fungus, bacterial and yeast cells. Animal cells include, but are not limited to insect, mammalian cells. Examples of mammalian cells include but are not limited to canine, feline, equine, bovine, porcine, rat, mouse, gerbil, guinea pig, human and non-human primates.

During the synthesis stage of the 3D matrix, for example, the MOSPs can be dispersed in a solution of a suitable polymer for the matrix. Then, the polymeric solution is transformed into the 3D matrix with the MOSPs embedded therein. Additionally, the transformation of the 3D matrix may be accomplished by, depending on the polymer, simply cooling the solution, for example, an agarose solution, by ionic crosslinking of alginate, by altering the pH of a solution containing poly-ionic molecules such as alginate and hyaluronic acid, or by covalently crosslinking other polymer molecules such as, for example, alginate or hyaluronic acid solution, by freeze-thawing a solution such as for PVA, or via other phase separating events such as solvent/non-solvent processing. Any of these semi-solid 3D matrices may be further lyophilized to create open pore 3D matrices. Additional modifications of the matrix include, but are not limited to spinning the polymers the matrix of hydrogel into fibers such that they can be readily assembled into or onto a three-dimensional structure.

The matrix itself may also be modified with bioaffecting molecules by adding the molecules in the solution of the suitable polymer. As used herein, "bioaffecting molecules" are molecules that affect cultured cells. For example, bioaffecting molecules can promote cell adhesion of cultured cells to the matrix. As an example, a bioaffecting molecule that promotes cells adhesion would include, but not be limited to, extracellular matrix (ECM) molecules, such as collagens, fibronectins, and laminins. Other examples of bioaffecting molecules include, but are not limited to, small molecule organics, peptides, chemotactic or cell-signaling molecules that are capable of affecting cell movement, cell growth, cell division, and/or cell differentiation. The MOSPs could be incorporated in or on the matrix, with or without bioaffecting molecules, and would thus serve as monitors for the state of cell growth, cell division, or cell differentiation, etc.

Indeed, placing the MOSPs in or on the matrix puts the MOSPs in close proximity to cells cultured within the matrix such that the MOSPs now may serve to monitor the respiratory state of the cells in real time. Cells can be seeded and grown on the matrix with no effect on cell growth. The luminescent property of the embedded MOSPs in a 3D matrix may be monitored, measured or detected optically in real time. For example, the change in fluorescent intensity of the MOSPs may be monitored by a microscope, such as, for example, a confocal microscope or an ELISA plate reader.

Thus, the MOSPs of the current invention are useful for monitoring oxygen utilization, production or consumption in a variety of settings including, but not limited to, cell culture, apoptosis assays, cell division assays and cell growth assays. Other uses of the MOSPs described herein include assays described in United States Patent Nos. 5,567,598 and 6,395,506, United States Published Application No. 2002/0192636A1 and United States Serial No. 09/966,505, all of which are hereby incorporated by reference in their entirety.

Additional uses include, but are not limited to using the oxygen sensors as part of a biosensor screening assay. Indeed, the MOSPs of the current invention can be interspersed within a matrix, as described herein, to provide a biosensor that is based upon cell metabolism. The 3D matrix comprising MOSPs could be used, for example, to screen drugs or toxins. Thus, in one embodiment of the current invention, the MOSPs on or in the 3D matrix would be used to monitor the oxygen consumption of cells in response to a particular stimulus, for example, a drug, toxin, virus, bacterial cell or other cell or compound. For example, culturing cells on the matrices of the current invention without the addition of any stimulus would establish a baseline oxygen utilization signature as measured by the amount of fluorescence produced by the MOSPs of the current invention. If the oxygen utilization signature were to change in response to a particular stimulus, then the change in the oxygen utilization signature would indicate a change in cellular metabolism. A change in the oxygen utilization signature would include an increase or decrease for any detectable amount. The biosensor assay could be used to test unknown agents, such as bioterrorism or chemical terrorism agents, or new or modified drug discovery compounds. The biosensors could be used high-throughput assays in any type of assay.

The matrices could, for example, be placed on a glass or plastic slide, a culture dish or flask or as part of an array. Thus, the current invention contemplates an array that can be used for testing more than one compound or stimulus simultaneously. For example, the array of the current invention can include one or more different type of 3D matrix. In drug discovery applications, for example, the array may comprise several matrices that would be suitable for a variety of different cell types. In such a setting, one drug, compound, toxin, etc. may be applied to the various cell types cultured on the array. The user would then monitor the oxygen utilization signatures to determine which cell types were vulnerable to the stimulus. Likewise, the array may consist of similar matrices such that the same cell types may be placed onto or into the matrix and different chemicals, compounds, toxins, or stimuli could simultaneously be applied to the cells to determine which stimuli had an effect on the cells. The array would be useful in a setting where genetic differences in cell types confer different rates of oxygen utilization, e.g., liver cells. Thus the array could be used to determine which genetic makeup of, for example, liver cells was more or less susceptible to a particular stimulus, based on metabolism as elucidated by the oxygen utilization signature.

Accordingly, the present invention provides a method for detecting oxygen in a sample, comprising measuring the intensity of the signal generated by the MOSPs for at least one time point. In one embodiment, the generated signal can be measured at multiple time points, i.e., more than one. Measurements taken at multiple time points can be compared to one another or compared to a baseline measurement as a way to monitor oxygen consumption or generation. For example, oxygen may be generated in certain chemical reactions that a user may wish to monitor. Similarly, the user may wish to monitor oxygen consumption for cultured cells at various time points, for example, in response to a particular stimulus.

As used herein, a sample can exist in or be derived from various environments. The sample may be a portion of the environment or the entire environment. The environments include, but are not limited to, aqueous or liquid environments such as a cell culture setting, water (for sensing water purity), concentrated air samples, or a chemical reaction, to which the MOSPs are directly added. Additional environments include but are not limited to, a cell culture 3D matrix, body fluid from an animal, lakes, rivers, oceans, public water supplies, etc. The environment may be *in vivo, in vitro* or *in situ.*

As used herein, the term "animal" is used to mean a vertebrate. In one embodiment, the vertebrate is a mammal. In another embodiment, the mammal is a human or non-human primate. The terms "subject", "patient" and "animal" are used interchangeably herein. Furthermore, as used herein, "body fluid" includes, but is not limited to, blood, plasma, serum, saliva, cerebrospinal fluid, synovial fluid, urine, bile and feces.

The present invention also provides for methods of making MOSPs comprising dispersing an oxygen-sensing particle and a hydrophobic coating material in a liquid wherein neither the hydrophobic coating nor the oxygen-sensing particle will readily dissolve, agitating the mixture and then removing the liquid from the mixture and drying the resulting powder. In one embodiment, carrier molecules may also be incorporated into the methods of making the MOSPs of the current invention.

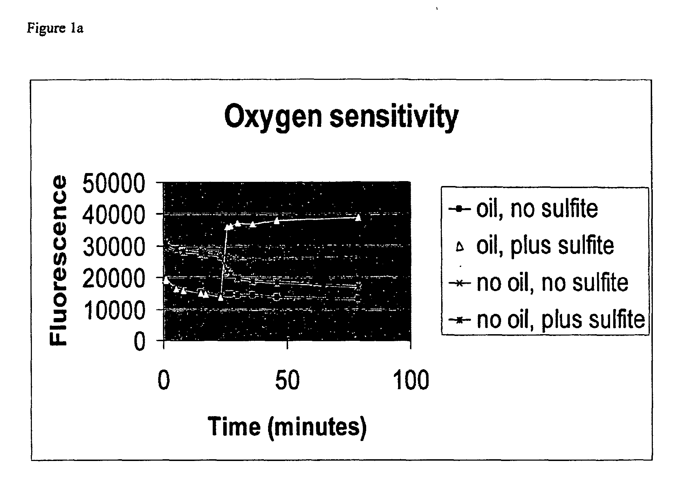

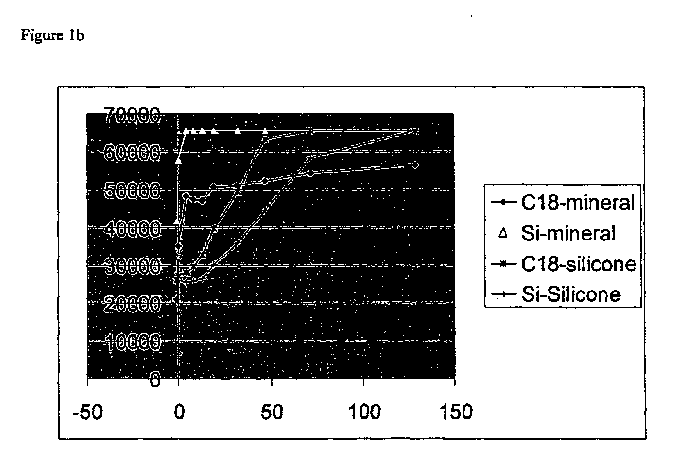

Figure 1 is a diagram showing the oxygen responses of the ruthenium dye-adsorbed silica gel particles to oxygen concentration change. In Figure 1 a the bead suspension was transferred by pipette into wells of a 96-well plate. 100 µl of water was added to each well and the fluorescence was measured. Then, 100 µl of 0.2M $NaSO_3$ aqueous solution was added to each well and the fluorescence was measured over time. Sodium sulfite reacted with oxygen to yield sodium sulfate, which reduced the local oxygen concentration resulting in increased fluorescent intensity. The data demonstrates that coating the beads with mineral oil preserved the oxygen responsiveness of the ruthenium dye-adsorbed silica gel particles. Figure 1b demonstrates that silica gel particles covered with covalently bonded hydrocarbon chains can be impregnated with oxygen-sensitive ruthenium dye to create particles responsive to oxygen without additional mineral oil treatment (blue curve). The response of these particles is faster than particles coated with silicone rubber (red). Note that the mineral oil-coated silica (yellow) also responds more rapidly than silicone rubber-coated particles (green).

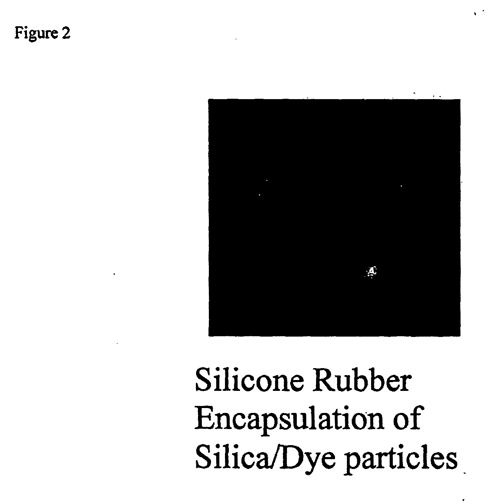

Figure 2 is a micrograph of silicone rubber-encapsulated oxygen-sensing particles.

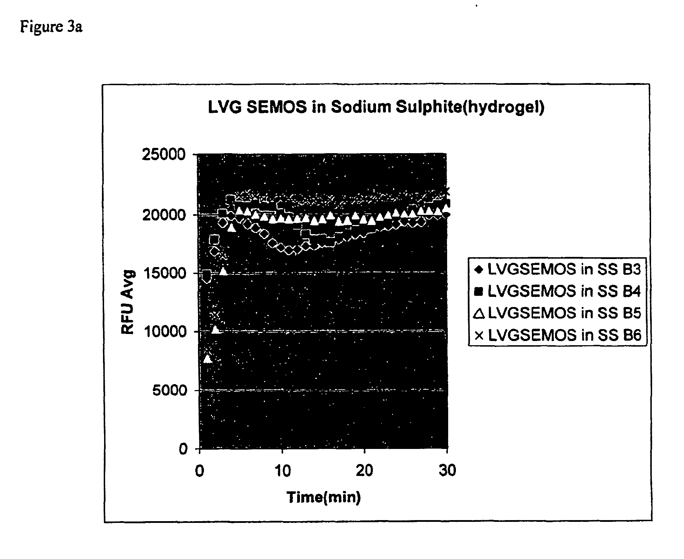

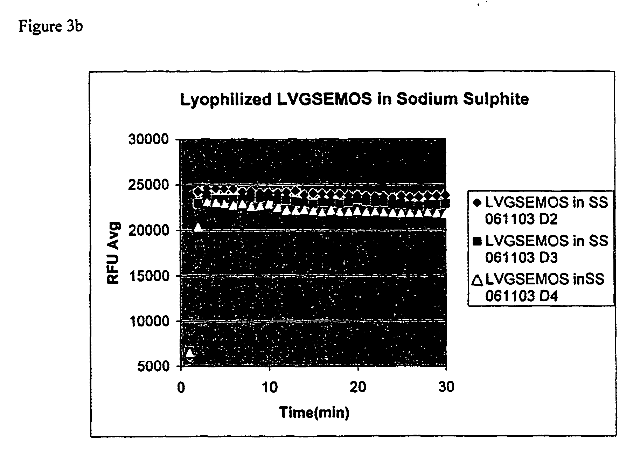

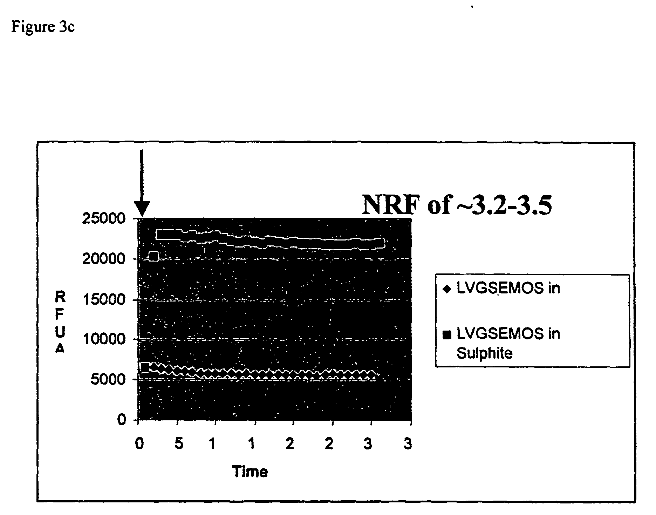

Figure 3 shows the responsiveness to changes in oxygen of the MOSPs of the current invention. Figure 3a shows the MOSPs embedded in a covalent alginate hydrogel and the dramatic change in fluorescence in the presence of sodium sulfite. Figure 3b shows that lyophilized hydrogels also demonstrate a very similar change in fluorescence, with even faster kinetics than the hydrogel-based matrix. Figure 3c shows the near immediate response of the MOSPs to the addition of sulfite, and this change in fluorescence would take several hours if performed in the standard PDMS polymer matrix. Thus, the kinetics are greatly enhanced due to embedding in hydrogels and lyophilized hydrogels.

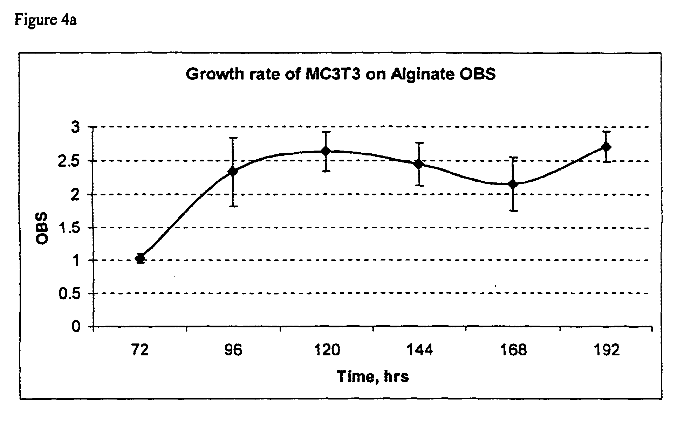

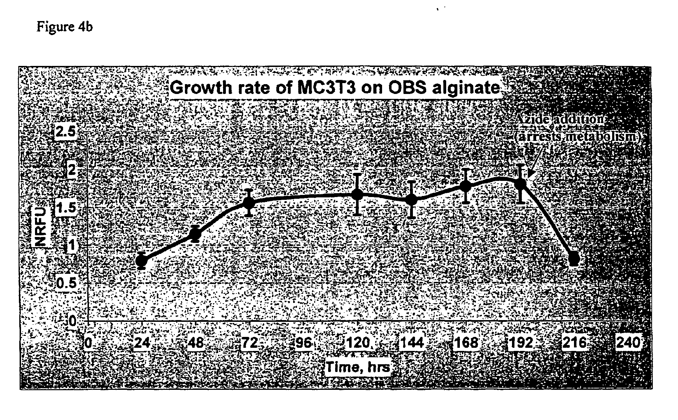

Figure 4 shows microencapsulated oxygen-sensing particles embedded within a 3D scaffold modified to support cell growth with fibronectin. MC3T3 osteoblasts cultured in 10% serum-containing medium previously demonstrated to support cell growth. This figure demonstrates that one can monitor cell growth and cell maintenance (quiescence), and that cellular metabolic alterations may be rapidly detected with the addition of a toxic substance (e.g., sodium azide) followed by a fluorescence read. Figure 4a shows the growth rate, as measured by fluorescence of MC3T3 cells. In Figure 4b, a known inhibitor of cellular metabolism was added to the MC3T3 culture at approximately 192 hours from time zero. The decrease in fluorescence thereafter demonstrates that the compositions of the current invention are useful in monitoring cellular metabolism.

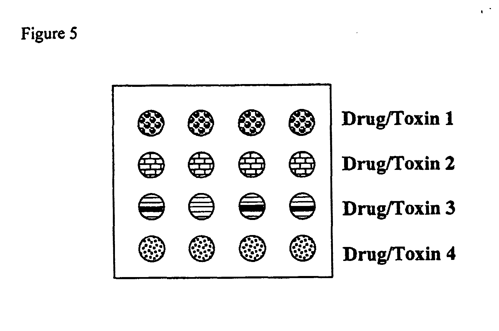

Figure 5 shows an example of a possible array comprising the compositions of the current invention. An array of scaffolds with oxygen-sensing capabilities and containing cells with some difference (different tumor types, different genetic make-ups, sensitive to different toxins) can be used to screen multiple drugs or drug candidates or, in the alternative, may be used to the response of screen different cell types to the same drug or drug candidate.

The examples presented herein are meant for illustrative purposes only and are not intended to limit the scope of the subject matter described herein.

*Examples*

*Example 1- Preparation of Oxygen-Sensing Particles on a* Carrier *(Polystyrene Beads)*

Part 1 - Preparation of Oxygen-Sensing Beads

Ruthenium dye crystals, ruthenium (II)-tris-(4,7-diphenyl-1,10-phenanthroline) diperchlorate (Ru(PDD)$_3$) (dye) on polystyrene beads were prepared as follows:

26.8 mg of polystyrene beads having a diameter of 105-125 µm (Polyscience) were weighed and suspended in 350 µl methanol. 18.06 mg of s-(4,7-diphenyl-1,10-phenanthroline) ruthenium (II) diperchlorate was weighed and added to 1 ml methanol to make 18 mg/l (stock A) and diluted in methanol at 1:5 (stock B) and 1:25 (stock C). 100 µl of bead suspension was mixed with stock A, B or C of the same volume to yield final concentrations of dye at 9 mg/ml, 1.8 mg/ml, and 0.4 mg/ml, respectively. The suspensions were left at 50°C with occasional mixing. The beads were then washed in methanol 1-2 times and then 2 times in water by microcentrifuge.

Part 2 - Detection of Oxygen Quenching on the Beads

To test response to oxygen, the bead suspension was transferred by pipette into wells of a 96-well plate. 100 µl of water was added to each well and the fluorescence was measured. Then, 100 µl of 0.2M $NaSO_3$ aqueous solution was added to each well and the fluorescence was measured over time. Sodium sulfite reacted with oxygen to yield sodium sulfate, which reduced the local oxygen concentration resulting in increased fluorescent intensity.

All beads with all 3 concentrations of dye crystals showed a modest increase in fluorescent signal over time; however, stock A, having 9 mg/ml dye crystal, showed the greatest response.

Part 3 - Observation of Oxygen Sensor Particles in Agarose Matrix

The sensor beads were further embedded in agarose in a fresh 96-well plate. First, the bead suspension was spun and water was removed to make a 50% suspension. 5 µl of suspension was added to each well of the 96-well plate. Hot or warm 0.8% agarose solution was prepared in PBS and 150 µl of the solution was added to the wells and mixed with the beads so that the beads were suspended in the solution. As the agarose solution cooled, pictures of the beads were taken on a Nikon confocal microscope using 488 nm excitation and red detector. The microscope was adjusted to focus through the z-axis to successfully sample the fluorescence.

*Example 2 - Preparation of Oxygen Sensor Particles on Silica Gel Beads*

Part 1 - Dye-adsorbed silica gel beads

As further described in U.S. Patents 5,567,598 and 6,395,506 (which are hereby incorporated by reference), $Ru(PDD)_3$ was adsorbed onto the silica gel particle by mixing the dye crystals (17 mg) with silica gel in about 400 µl water. A series of dilutions were made at 1:2 and 1:5 by mixing the dye-adsorbed silica gel bead suspension with water.

Part 2 - Detection of oxygen quenching on the beads

100 µl of 0.2 M sodium sulfite was added to reduce oxygen concentration in the wells. A change of fluorescence intensity was observed. All wells had strong fluorescent signals under BMG fluorometer (37°C).

Part 3 - Comparison of $Ru(PDD)_3$ silica gel particles and polystyrene particles

$Ru(PDD)_3$ adsorbed silica gel particles and polystyrene particles 100 µl were added to wells of a 96-well plate. The beads were continuously observed under 172 Nikon confocal microscope. The silica beads showed brighter fluorescence than the polystyrene beads.

*Example 3 - Preparation of Dye-Adsorbed Silica Beads Embedded in Silicone Rubber*

The dye-adsorbed silica gel beads (27.5 mg) prepared from Example 2 were added to a 25 ml round bottom flask. Into a 50 ml beaker, a mixed stock of silicone in methylene chloride was prepared. The mixed silicone stock was prepared from 2 parts of GE 1893B heat-cure silicone and 2 parts of GE 1893A heat-cure silicone (polydimethyl siloxane (PDMS)). In a fume hood, 7 ml methylene chloride was added to the beaker to obtain a concentration of 110 mg/ml silicone mixture.

50 µl of the silicone mixture, which contained 5.5 mg silicone, was mixed with the dye-adsorbed silica gel beads. The mixture was rotary evaporated to dryness. Another 200 µl of silicone methylene chloride mixture was added and the mixture was evaporated to dryness at 70°C for about 1 hour and removed to room temperature. The dye-adsorbed silica gel beads were embedded in a thin layer of silicone rubber.

The embedded beads showed strong increase in fluorescence in response to addition of sodium sulfite and decreased oxygen tension. The significant increase in fluorescent intensity occurred within about 10 minutes, and continued over a time course. In contrast, the dye-adsorbed beads which were not embedded in silicone did not show the response, i.e., the increase of fluorescent intensity, to oxygen concentration change, even though the initial fluorescent intensity was comparable to that of the embedded beads. The results demonstrate that silicone rubber aided the response of the dye-adsorbed beads to oxygen concentration change.

*Example 4 - Preparation of Dye-Adsorbed Polystyrene Beads Embedded in Silicone Rubber*

The dye-adsorbed polystyrene beads stock A from Example 1 were suspended in 200 µl water. The suspension was added to 2 microfuge tubes (100 µl each) and speed-vacuumed to dryness. One tube of the dried beads was resuspended in 100 µl water, from which 25 µl was added to each well of a flat bottom 96-well plate. The other tube of dried beads was resuspended in 100 µl Loctite Special RTV Silicone (Loctite Corporation), from which 25 µl was added to each well of the flat bottom 96-well plate.

Fluorescence was observed pre-polymerization and post-polymerization of the silicone rubber under BMG fluorometer at a 37°C, 90% humidity, and tissue culture incubator. 100 µl of water was added to each well and fluorescence was measured at time zero. 100 µl 0.2 M sodium sulfite was added so that the final concentration in the well was about 0.1 M. Fluorescent intensity was measured overtime.

The silicone-embedded polystyrene beads showed a very slight increase in fluorescent intensity in response to addition of sodium sulfite and decreased oxygen tension, as compared with the silicone-embedded polystyrene beads without the addition of sulfite. In contrast, the dye-adsorbed polystyrene beads that were not embedded showed no increase in fluorescent intensity at all, but showed a decrease in the fluorescent intensity.

Silicone rubber was vital to the response of the dye-adsorbed polystyrene beads to oxygen concentration change. This example demonstrates the necessity of a silicone rubber, or rather, hydrophobic coating.

*Example 5 - Preparation of Dye-Adsorbed $C_{18}$ Silica Beads*

$C_{18}$ hydrocarbon chain covalently attached Baker Bond™ silica (J.T. Baker, Inc., Phillisburg, NJ) 0.61 g was added to a 25 ml round bottom flask. Ru(PDD)$_3$ (GFS Chemical, Inc., Powel, OH) (0.61 g) was added to the 25 ml round bottom flask. Ru(PDD)$_3$. 5.6 mg and methylene chloride 10 ml were added to the flask and left at room temperature overnight. Ru(PDD)$_3$ dyes impregnated the modified silica gel beads in the methylene chloride. The mixture was then dried by rotary evaporation to obtain orange powder beads. The beads were placed in microfuge tubes and wetted with 5 µl 10% Triton X-100 and 200 µl water.

The solution containing the beads was diluted 1:5 and put in wells of a 96-well plate containing 100 µl of water. Sulfite 0.1 ml (0.2 M) was added and the beads were observed for change in fluorescence as compared to dye-adsorbed uncoated silica beads. $C_{18}$ covalently attached silica beads showed a modest signal increase within 10 minutes of addition of sulfite and showed stronger response over a longer period of time than the uncoated dye-adsorbed silica particles. In the meantime, the addition of water instead of sulfite caused the fluorescent intensity to slightly decrease. Thus, the $C_{18}$ covalently attached silica beads were useful as oxygen sensor particles by themselves.

*Example 6 - Effect of Mineral Oil on Dye Response*

Light mineral oil (100 µl) was added to wells of a 96-well plate which contained small amount of either dye-adsorbed silica gel beads as prepared from Example 2 or the dye adsorbed $C_{18}$ covalently attached silica gel beads as prepared from Example 5. As a comparison, silicone rubber (PDMS) (100 µl) was also added to wells containing small amounts of either dye-adsorbed silica gel or dye adsorbed $C_{18}$ covalently attached silica gel beads.

Then, 100 µl 0.1 M sodium sulfite or water was added to each well, and fluorescence was measured at time zero and over a time course. Particles encapsulated with mineral oil showed fast response and significantly increased intensity than those covered in PDMS. Even the dye-adsorbed silica gel beads without $C_{18}$ coating showed fast response to oxygen concentration change (data not shown). As observed under the microscope, there was a thin layer of mineral oil remained associated with the beads.

*Example 7 - Encapsulation of Oxygen-Sensing Particles*

In a 100-ml glass beaker equipped with a cross-shape magnetic stirring bar was added 50 ml of acetone (J. T. Baker, HPLC grade), 1.0 gm of oxygen-sensing particles (tris(4,7-diphenyl-10-phenanthroline) ruthenium dichloride pentahydrate) that were pre-attached to a silica carrier molecule, 1.0 gm of BASF Masil SF-201 polymer containing 1000ppm of platinum catalyst (Gelest, cat# 38-2501) and 50ppm of polyvinylmethylsiloxane (UCT, P6925-KG) inhibitor. While the contents of the beaker was under vigorous stirring, 0.2ml of BASF Masil XL-1 crosslinker was introduced into the beaker, via a syringe, in one portion. The beaker was then covered with an aluminum foil and stirred for one hour at ambient room temperature. The magnetic stirrer was then retrieved from the beaker and most of the acetone was removed by pipetting. The resulting orange "wet" residue was left in the beaker and placed inside a fume hood until dry. The orange-yellow loose powder (MOSP) was thus obtained. The weight of the loose powder (MOS) was 1.75 gm.

*Example 8 - Comparison of MOSPs with Unencapsulated Oxygen-Sensing Particles*

Agar (1.5% in hot water) (Grade A) was made to host the MOSPs. To Becton, Dickinson and Company MGIT (Mycobacterial Growth Indicator Tubes) COC plastics tubes (Becton, Dickinson and Company, Franklin Lakes, USA) were added 0.01 gm of MOSP (Example 2) or unencapsulated oxygen-sensing particles (tris(4,7-diphenyl-10-phenanthroline) ruthenium dichloride pentahydrate) followed by four drops (~0.05ml) of hot Agar solution. Upon cooling, a gel was formed in each tube while MOS or unencapsulated oxygen-sensing particles were trapped under the gel. All tubes were sealed with Hungate caps. The tubes were then evacuated through a syringe needle connected to a vacuum pump. Next, the generated signals in each tube were measured with a reader (Firefox) used for QC and R&D for similar studies. After completion the vacuum signal measurements, caps were opened to discharge the vacuum tubes and the intensity of the generated signal was measured again in the presence of atmospheric air. Table 1 shows that the Dynamic Range of the MOSP tubes are shown to be 10 to 20 times better than that of the tubes containing the unencapsulated oxygen-sensing particles.

Table 1

| Tube | With MOS #1 | With MOS #2 | With VRAST # 3 | With VRAST #4 |
|---|---|---|---|---|
| Vacuum signal (mV) | 6.880 | 6.880 | 2.670 | 3.200 |
| Air signal (mV) | 0.816 | 0.819 | 1.982 | 1.874 |
| Dynamic Range: (Vac - Air)/Air | 7.43 | 7.40 | .035 | .71 |

*Example 9 - Making Mineral Oil-Coated Oxygen Sensor Particles*

Dye-adsorbed silica gel was added to each of 2 microfuge tubes. Mineral oil (200 µl) was added to one tube and water (200 µl) to the other. The tubes were vortexed and microfuged. Supernatant was removed and the tubes were spanned again to remove residual liquid. The beads were suspended in 250 µl water and aliquoted at 100 µl into each well of a 96-well plate for measuring of fluorescence change.

Either 100 µl water or 0.5 M sodium sulfite was added to each well to observe response. As shown in Figure 1, the mineral oil coated dye-adsorbed silica beads showed significant response to addition of sulfite and reduction of oxygen concentration, while water-coated dye-adsorbed silica bead showed a decrease in intensity with addition of sulfite, which was useless for detecting oxygen concentration.

*Example 10 - Testing Oxygen Sensor Particles in Agarose Scaffold*

Beads prepared from Example 9 were also added to 250 µl 1% hot agarose/PBS solution, respectively. The agarose/PBS suspension (100 µl) was added to flat bottom of well of a 96-well plate. After agarose cooled, 100 µl water or 0.5 M sodium sulfite was added to each well for measuring fluorescence over time.

As shown in Figure 3, despite a decrease in signal over the initial 5 minutes, mineral oil-coated, dye-adsorbed silica beads showed significant response to addition of sulfite and reduction of oxygen concentration, and thus, was highly responsive to change in oxygen concentration, while water-coated, dye-adsorbed silica bead showed a decrease in intensity with addition of sulfite, which was useless for detecting oxygen concentration. The overall response time was slower than that in wells without agarose.

*Example 11 - Making Silicone-Coated Naked Dye Particles*

Silicone rubber spheres containing naked dye crystal particle (without adsorption or any form of attachment to a carrier bead such as silica gel bead or polystyrene bead) were prepared by sonicating dye particles in silicone and a nonsolvent of silicone such as water. The sonication created an emulsion of silicone in water, where the spheres polymerized and encapsulated individual dye crystals. The single dye crystal particle, coated with a thin layer of silicone rubber, was approximately 50 µm in diameter. The coated dye was useful for embedding within cell culture scaffold.

*Example 12 - Making Oxygen Sensor Particle-Containing Scaffold for Cell Culture*

Dye-adsorbed silica particles coated with mineral oil in suspension were mixed with water at 1:1. Diluted suspension (50 µl) was added to 2 ml alginate (MVG alginate, ProNova, Norway) solution 2% (w/v), which had been obtained by slowly dissolving alginates in 0.1 M MES buffer (pH 6.0). Suitable amount of hydroxyl benzotiazole (HoBt, H-2006, Sigma) and Adipic Acid Dihydrate (AAD, MW 174) were added for crosslinking. 1-ethyl-3-(3-dimethyl aminopropyl) carbodiimide (EDC, MW 191.7, Pierce) was quickly added to initiate crosslinking reaction in the polystyrene dishes. The crosslinking reaction last for 2 hours. The oxygen sensor particles were well dispersed in the crosslinked hydrogel. The hydrogel was cut out to obtain several 5 mm x 1 mm disks, washed, lyophilized, and dried to obtain the scaffolds for cell culture.

The scaffolds were further coated with collagen I solution 100 µg/ml and washed in PBS and water and lyophilized again. The resultant scaffold was seeded with MC3T3 cells at 100,000 cells/scaffold and cultured for 2 days with 10% serum-containing medium. Cells were stained and observed under confocal microscope.

*Example 13 - Making ECM-Modified Scaffold Having Sensor Particles*

As in Example 12, instead of collagen I solution, the scaffolds were soaked with ECM solution 100 µg/ml containing laminin, fibronectin, collagen IV, and collage I, respectively. The same steps were followed to obtain modified scaffold and seeded with MC3T3 cells. Sulfite at 200 mM was also added to quench the oxygen sensor particles observed under confocal microscope over 16 hours.

**Claims**

1. A composition for detecting and monitoring oxygen in a sample comprising:

   a) a core comprising at least one oxygen-sensing particle; and

   b) a hydrophobic coating material surrounding said core.

2. The composition of claim 1, wherein said at least one oxygen-sensing particle is luminescent and is selected from the group consisting of a tris-4,7-diphenyl-1,10-phenanthroline ruthenium (II) salt, a tris-2,2'-bipyridyl-ruthenium (II) salt, a tris-1,7-diphenyl-1,10 phenanthroline ruthenium (II) salt, 9,10-diphenyl anthracene, platinum (II) octaethyl porphyrin complexes and palladium (II) octaethyl porphyrin complexes, palladium-meso-tetra(4-carboxyphenyl) porphine, palladium-meso-tetra(4-carboxyphenyl) porphyrin dendrimer and palladium-meso-tetra(4-carboxyphenyl) tetrabenzoporphyrin dendrimer, and further, wherein said tris-4,7-diphenyl-1,10-phenanthroline ruthenium (II) salt is selected from the group consisting of tris-4,7-diphenyl-1,10-phenanthroline ruthenium (II) dichloride pentahydrate, tris-4,7-diphenyl-1,10-phenanthroline ruthenium (III) trichloride, tris-4,7-diphenyl-1,10-phenanthroline ruthenium (II) diperchlorate and tris-4,7-diphenyl-1,10-phenanthroline ruthenium hexafluorophosphate.

3. The composition of claim 1, wherein said hydrophobic coating material comprises a polymer selected from the group consisting of a functionalized polydimethylsiloxane, silicone rubber, polytetrafluoroethylene (PTFE), polysterene, and mineral oil, and further, wherein said functional polydimethylsiloxane is selected from the group consisting of vinyl functionalized polydimethylsiloxanes, hydrido functionalized polydimethylsiloxanes, alkoxyl functionalized polydimethylsiloxanes and acetoxyl functionalized polydimethylsiloxanes.

4. The composition of claim 1, further compromising a three-dimensional hydrogel matrix wherein said hydrogel is selected from the group consisting of ionically crosslinked agarose, ionically crosslinked alginate, modified alginate hyaluronic acid, modified hyaluronic acid, polyacrylimide, polyethylene glucose (PEG), polyvinylalcohol (PVA), poly methylmethacrylate (PMMA), collagen and combinations thereof.

5. A method of detecting the oxygen content in a sample for at least one time point, comprising the composition of claim 1.

6. The method of claim 5, wherein said at least one time point comprises a first and second time point, and further comprising comparing said detected levels at said first and second time points.

7. A method of making the composition of claim 1, comprising:

   a) dispersing said oxygen-sensing particle in a liquid, wherein said oxygen-sensing particle does not dissolve in said liquid;

   b) dispersing said hydrophobic coating material in said liquid;

   c) agitating said liquid that contains said oxygen-sensing particle and said hydrophobic coating material;

   d) removing said liquid after said agitation; and

   e) drying the resulting powder after said liquid removal.

8. A method of detecting the oxygen content in a sample for at least one time point, comprising the composition of claim 4.

9. A method of screening the cellular metabolic effect of a compound comprising the compound of claim 1.

10. A method of screening the cellular metabolic effect of a compound comprising the compound of claim 4.

Figure 1a

Figure 1b

Figure 2

Silicone Rubber
Encapsulation of
Silica/Dye particles

Figure 3a

Figure 3b

Figure 3c

Figure 4a

Growth rate of MC3T3 on Alginate OBS

Figure 4b

Growth rate of MC3T3 on OBS alginate

Figure 5

Drug/Toxin 1

Drug/Toxin 2

Drug/Toxin 3

Drug/Toxin 4

EP 1 510 817 A1

| European Patent Office | EUROPEAN SEARCH REPORT | Application Number EP 04 01 8192 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | YONG-EUN LEE KOO ET AL: "REAL-TIME MEASUREMENTS OF DISSOLVED OXYGEN INSIDE LIVE CELLS BY ORGANICALLY MODIFIED SILICATE FLUORESCENT NANOSENSORS" ANALYTICAL CHEMISTRY, AMERICAN CHEMICAL SOCIETY. COLUMBUS, US, vol. 76, no. 9, 1 May 2004 (2004-05-01), pages 2498-2505, XP001196749 ISSN: 0003-2700 * page 2501; figure 1 * | 1-3,5-9 | G01N31/22 G01N21/64 |
| X | COLLINSON M M: "Recent trends in analytical applications of organically modified silicate materials" TRAC, TRENDS IN ANALYTICAL CHEMISTRY, ANALYTICAL CHEMISTRY. CAMBRIDGE, GB, vol. 21, no. 1, January 2002 (2002-01), pages 31-39, XP004334859 ISSN: 0165-9936 * pages 31-35 * | 1-9 | |
| X | MCNAMARA K P ET AL: "DYE-ENCAPSULATING LIPOSOMES AS FLUORESCENCE-BASED OXYGEN NANOSENSORS" ANALYTICAL CHEMISTRY, AMERICAN CHEMICAL SOCIETY. COLUMBUS, US, vol. 70, no. 22, 15 November 1998 (1998-11-15), pages 4853-4859, XP000799209 ISSN: 0003-2700 * page 4854 - page 4855 * * page 4857, right-hand column * | 1,2,5-9 | TECHNICAL FIELDS SEARCHED (Int.Cl.7) G01N |

-/--

The present search report has been drawn up for all claims

| Place of search | Date of completion of the search | Examiner |
|---|---|---|
| Munich | 20 December 2004 | Michalitsch, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

21

**European Patent Office**

# EUROPEAN SEARCH REPORT

Application Number

EP 04 01 8192

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CLARK H A ET AL: "Subcellular optochemical nanobiosensors: probes encapsulated by biologically localised embedding (PEBBLEs)" SENSORS AND ACTUATORS B, ELSEVIER SEQUOIA S.A., LAUSANNE, CH, vol. 51, no. 1-3, 31 August 1998 (1998-08-31), pages 12-16, XP004153984 ISSN: 0925-4005 * page 13 - page 15, left-hand column * | 1,2,5-9 | |
| X | LUEBBERS D W ET AL: "NANOENCAPSULATED FLUORESCENCE INDICATOR MOLECULES MEASURING PH AND PO2 DOWN TO SUBMICROSCOPICAL REGIONS ON THE BASIS OF THE OPTODE-PRINCIPLE" ZEITSCHRIFT FUER NATURFORSCHUNG. SECTION C. BIOSCIENCES, XX, XX, vol. 32C, no. 133, 1977, pages 133-136, XP000653777 ISSN: 0341-0382 * the whole document * | 1 | |
| P,A | BROWN J Q ET AL: "Fabrication and deployment of nanoscale fluorescent intracellular probes" PROCEEDINGS OF THE 25TH. ANNUAL INTERNATIONAL CONFERENCE OF THE IEEE ENGINEERING IN MEDICINE AND BIOLOGY SOCIETY. CANCUN, MEXICO, SEPT. 17, vol. VOL. 4 OF 4. CONF. 25, 17 September 2003 (2003-09-17), pages 3384-3387, XP010693993 ISBN: 0-7803-7789-3 * page 3384, right-hand column - page 3385, left-hand column; figures 1-3 * | 1-4 | |

TECHNICAL FIELDS SEARCHED (Int.Cl.7)

The present search report has been drawn up for all claims

| Place of search | Date of completion of the search | Examiner |
|---|---|---|
| Munich | 20 December 2004 | Michalitsch, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)